# FASCICULE DE BREVET EUROPEEN

(11) **EP 1 073 414 B1**
(45) Date de publication et mention de la délivrance du brevet: **26.06.2002**
(21) Numéro de dépôt: 99919243.8
(22) Date de dépôt: 16.04.1999
(51) Int. Cl.: A61K 9/10, A61K 47/12, A61K 47/24

(54) **UTILISATION DE COMPOSITIONS PHARMACEUTIQUES GELIFIABLES EN PARODONTOLOGIE**
VERWENDUNG GELIERBARER PHARMAZEUTISCHER ZUSAMMENSETZUNGEN IN DER PARODONTOLOGIE
USE OF PHARMACEUTICAL COMPOSITIONS CAPABLE OF BEING GELLED IN PERIODONTOLOGY

(30) Priorité: 30.04.1998 BE 9800329
(43) Date de publication de la demande: 07.02.2001
(73) Titulaire: UCB, S.A., 1070 Bruxelles (BE)
(72) Inventeur: FANARA, Domenico, B-4520 Wanze (BE); VRANCKX, Henry, B-1190 Bruxelles (BE); DELEERS, Michel, B-1630 Linkebeek (BE); GROGNET, Pierre, B-1070 Bruxelles (BE)
(74) Mandataire: Lechien, Monique
(86) Numéro de dépôt international: EP9902552
(87) Numéro de publication internationale: WO9956726

(56) Documents cités:
- EP-A- 0 429 224
- EP-A- 0 550 960
- WO-A-94/10978

## Description

La présente invention se rapporte à des compositions pharmaceutiques permettant la libération prolongée d'au moins une substance active, à des procédés de préparation de ces compositions, ainsi qu'à leur utilisation pour le traitement des parodontites, gingivites, abcès dentaires, aphtes et mycoses.

Les parodontites sont des maladies qui se traduisent par la destruction des tissus de soutien des dents suite à une inflammation causée par les microorganismes anaérobies de la plaque dentaire. Sans traitement, la maladie évolue inéluctablement et des populations bactériennes de plus en plus nombreuses ne cessent d'entretenir la réaction immunitaire qui déclenche les phénomènes de paradontolyse et de paradontoclasie. Dès qu'il atteint une profondeur supérieure à 3 mm, le sillon gingivo-dentaire est considéré comme une poche parodontale. Les parodontites s'accompagnent également de la résorption de l'os alvéolaire. Leur évolution conduit finalement à la perte de dents.

Les parodontites sont des maladies qui touchent plus fortement la population adulte de plus de quarante ans. Les parodontites ne sont cependant pas des maladies des personnes âgées; elles atteignent assez fréquemment les jeunes.

On peut distinguer les infections aiguës (gingivite superficielle ou abcès parodontaux profonds); les infections "rapidement évolutives" (parodontite de l'adolescent et du jeune adulte); enfin les infections chroniques évoluant par paliers (maladie parodontale chronique).

Les méthodes de traitement des parodontites comprennent notamment l'hygiène quotidienne, les moyens mécaniques (nettoyages, détartrages, surfaçage des racines, ...) ainsi que les moyens antiseptiques et antibiotiques locaux et systémiques.

Les antiseptiques peuvent être administrés localement sous forme de bains de bouche, de dentifrices, de crèmes. Toutefois, ce mode d'administration locale ne permet pas aux antiseptiques de diffuser jusqu'au fond des poches parodontales. La fonction de ce mode d'administration ne peut dépasser le stade de la prophylaxie.

Quant aux antibiotiques, ils ne sont pas d'avantage actifs en administration topique pour la même raison, puisqu'ils ne parviennent pas à atteindre les biofilms bactériens (ou plaque) dans leur profondeur. Lorsque la sévérité du cas le nécessite, les antibiotiques sont donc généralement administrés par voie systémique, escomptant leur diffusion jusqu'aux poches parodontales à partir du fluide gingival qui ne cesse de s'y déverser.

Une autre méthode consiste à utiliser des compositions pharmaceutiques permettant la libération contrôlée d'agents antiseptiques ou antibiotiques, insérées directement au fond de la poche parodontale. Il existe des compositions à libération prolongée déposées sur des dispositifs solides, tels que fils imbibés, écailles de gélatine, .... De tels dispositifs peuvent laisser sur place des trames insolubles qui doivent bien entendu être retirés en fin de traitement.

La littérature mentionne également des compositions pharmaceutiques fluides, telles que suspensions ou émulsions plus ou moins visqueuses qui sont administrées dans la poche parodontale, généralement à l'aide de seringues.

La demande de brevet internationale WO 95/34287 décrit des compositions lipidiques biodégradables sous la forme de phases cristallines L2, permettant la libération contrôlée de substances actives et comprenant, outre la substance active, au moins un diacyl glycérol d'acide gras insaturé ayant de 16 à 22 atomes de carbones ou d'acide gras saturé ayant de 14 à 22 atomes de carbone, et au moins un phospholipide choisi parmi les glycérophosphatides et sphingophosphatides, et, éventuellement, au moins un liquide polaire choisi parmi l'eau, le glycérol, l'éthylène glycol et le propylène glycol. Ces compositions possèdent la particularité de se transformer au contact de l'eau en phases cristallines liquides cubiques, ce qui permet de "mouler" la substance active dans le site où l'on désire que l'action ait lieu. Ce document mentionne, parmi d'autres applications, la possibilité d'utiliser de telles compositions pour le traitement de la parodontite. Toutefois, l'efficacité de telles compositions dans le traitement de la parodontite n'est pas illustrée dans ce document.

Le brevet européen 429224 décrit des compositions se présentant sous la forme de gels contenant de 1 à 99% en poids de monooléine et de 1 à 90% en poids de substance active, que l'on place dans la cavité parodontale. En présence de l'eau environnante, ces compositions deviennent plus visqueuses et maintiennent la substance active près de son site d'action. La substance active est libérée lentement de manière contrôlée.

Le brevet US 5230895 décrit l'utilisation de compositions se présentant sous la forme de solutions ou de pâtes capables de se transformer en gel lorsqu'elles ont été placées dans la poche parodontale. Ces compositions sont biodégradables et permettent la libération contrôlée de la substance active, dans le site d'action. Elles contiennent un mélange de glycérides et d'une substance active choisi de manière telle qu'il soit capable de former un gel dans l'environnement de la poche parodontale. Les compositions illustrées dans ce document contiennent au moins 70% de Myverol™ 18-92 qui est une composition de monoglycérides de tournesol ayant une teneur en monoglycéride d'au moins 90%.

Le brevet US 5143934 décrit des compositions permettant l'administration par libération contrôlée d'une substance active dans une poche parodontale, comprenant au moins un monoglycéride et au moins une huile végétale dans des proportions suffisantes pour former une phase cristalline liquide au contact de l'eau présente dans la poche parodontale. Ces compositions sont solides à température ambiante, mais elles ont un point de fusion inférieur à la température corporelle.

Dans ce document les résultats obtenus pour plusieurs compositions de ce type sont comparés avec les résultats obtenus par un traitement conventionnel mécanique. On constate que les compositions qui y sont décrites permettent d'obtenir une réduction de la taille des poches parodontales et une diminution des saignements pendant trois mois de traitement. Toutefois, l'effet obtenu par l'application des compositions n'est pas meilleur, et est même légèrement inférieur, au résultat que l'on peut obtenir avec le traitement mécanique de référence.

En outre, puisque les compositions décrites dans le brevet US 5143934 sont solides à température ambiante, elles doivent être liquéfiées au moment de l'insertion dans la poche parodontale.

La demanderesse vient maintenant de découvrir de nouvelles compositions pharmaceutiques, facilement applicables dans la poche parodontales et permettant la libération prolongée de substances actives dans cette poche. La demanderesse vient en particulier de découvrir que ces nouvelles compositions se prêtent très bien au traitement de la parodontite, tant par leur efficacité que par la facilité de leur utilisation. En outre, ces compositions sont obtenues par un procédé de préparation extrêmement simple.

C'est pourquoi la présente invention concerne des compositions pharmaceutiques fluides permettant la libération contrôlée d'au moins une substance active comprenant
a) une quantité thérapeutiquement efficace d'au moins une substance active,
b) de 3 à 55% en poids de phospholipide,
c) de 16 à 72% en poids de solvant pharmaceutiquement acceptable, et
d) de 4 à 52% en poids d'acide gras,
ces compositions ayant la propriété de se gélifier instantanément en présence d'une phase aqueuse.

Selon un autre aspect, l'invention se rapporte à des procédés pour la préparation de ces compositions.

Les compositions selon la présente invention comprennent une quantité thérapeutiquement efficace d'au moins une substance active. Cette dernière peut être liposoluble ou hydrosoluble. On citera à titre d'exemple des antibiotiques, en particulier les antibiotiques actifs contre les bactéries anaérobies tels que la doxycycline ou la minocycline et leurs sels pharmaceutiquement acceptables, des agents anti-infectieux, tels que le métronidazole, la chlorhexidine, le chlorure de benzalkonium, le p-chloro-m-crésol, l'alcool 1,2-dichlorobenzylique, l'hexamidine ou le chlorofène et leurs sels pharmaceutiquement acceptables, des anesthésiques locaux tels que la lidocaïne, la procaïne, la tétracaïne, l'articaïne, la bupivacaïne, la mépivacaïne ou la prilocaïne et leurs sels pharmaceutiquement acceptables, des anti-inflammatoires stéroïdiens ou autres, tels que l'hydrocortisone, la cortisone, la prednisone, la prednisolone, la méthylprednisolone, la triamcinolone, la bétaméthasone ou la dexaméthasone et leurs sels pharmaceutiquement acceptables ainsi que l'acéclofénac, le diclofénac, l'ibuprofène et le piroxicam et leurs sels pharmaceutiquement acceptables, des antimycosiques tels que la griséofulvine, l'amphotéricine B, la natamycine, la nystatine et leurs sels pharmaceutiquement acceptables ou encore des substances actives peptidiques telles que la calcitonine, la somatostatine, la bone growth hormone et autres facteurs de croissance ou de réparation.

Les compositions selon la présente invention contiennent de 3 à 55% de phospholipide. Les phospholipides utilisables selon la présente invention sont des esters phosphoriques de polyols et d'acides gras. Ils peuvent provenir de sources très variées, tant naturelles que par voie de synthèse. Les phospholipides peuvent être hydrogénés ou non hydrogénés. On citera à titre d'exemples, la phosphatidylcholine, la phosphatidylcholine hydrogénée, les sels du phosphatidylglycérol, la dicaproylphosphatidylcholine ou les sels du distéaroylphosphatidylglycérol. On peut également utiliser ces phospholipides en mélange. De préférence, le phospholipide présent dans les compositions selon la présente invention est la phosphatidylcholine.

Lorsque le phospholipide est choisi parmi la phosphatidylcholine, les sels du phosphatidylglycérol, la dicaproylphosphatidylcholine ou les sels du distéaroylphosphatidylglycérol, les compositions préférées selon la présente invention contiennent de 15 à 55% en poids de phospholipide. Lorsque le phospholipide est une phosphatidylcholine hydrogénée, les compositions selon la présente invention contiennent de 3 à 11%, de préférence de 3 à 10% en poids de phospholipide.

Les compositions selon la présente invention contiennent un ou plusieurs solvants pharmaceutiquement acceptables. Par solvant pharmaceutiquement acceptable, on entend un solvant tel que le propylène glycol, les polyéthylène glycols, les huiles minérales telles que l'huile de paraffine ou les huiles de silicone ou tout autre solvant dans lequel le phospholide utilisé est soluble. On peut également utiliser des mélanges de plusieurs solvants pharmaceutiquement acceptables. On utilise de préférence le propylène glycol. Le solvant utilisé est pharmaceutiquement acceptable, ce qui signifie que le solvant ne produira pas de réaction biologique se traduisant par des infections, inflammations ou autres phénomènes de rejet.

Les compositions selon la présente invention contiennent également de 4 à 52% d'au moins un acide gras. Les acides gras utilisables selon la présente invention sont des acides organiques carboxyliques saturés ou insaturés contenant de 4 à 22 atomes de carbone, de préférence de 8 à 18 atomes de carbone. A titre d'exemple, on citera l'acide oléique, l'acide caprylique, l'acide caprique, l'acide caproïque, l'acide myristique, l'acide butyrique... On peut également utiliser des mélanges d'acides gras. L'acide gras préféré selon la présente invention est l'acide oléique.

Eventuellement, les compositions selon la présente invention peuvent aussi contenir jusqu'à 15% en poids d'eau. On notera que la quantité d'eau présente dans les compositions selon l'invention est choisie de manière à ce que la composition ait la consistance désirée pour l'application envisagée.

La demanderesse a également découvert que des phospholipides se présentant sous la forme de mélanges commerciaux conviennent pour les compositions selon la présente invention. Comme exemple de telles compositions commerciales, on citera Phosal 50 PG™ (55,8% de phosphatidylcholine, 1,9% d'acides gras de soja, 2,9% de monoglycérides de tournesol, 1,9% d'éthanol, 37,3% de propylène glycol, 0,2% de palmitate d'ascorbyle), Phosal 53 MCT™ (60,8% de phosphatidylcholine, 2% acide oléique, 3% de monoglycérides de tournesol, 5% d'éthanol, 29% de triglycérides, 0,2% de palmitate d'ascorbyle), disponibles chez NATTERMANN PHOSPHOLIPID GmbH.

Les compositions selon la présente invention peuvent également contenir les composants optionnels suivants: jusqu'à 5% en poids de monoglycéride ou de diglycéride ou d'un mélange de mono- et de diglycéride et/ou jusqu'à 15% en poids de triglycérides.

Les compositions selon la présente invention peuvent en outre contenir un ou plusieurs agents conservateurs (tel que l'éthanol), un ou plusieurs agents antioxydants (tel que le palmitate d'ascorbyle) ou un ou plusieurs agents complexants (tel que l'EDTA (éthylènediamine tétraacétate)).

Les compositions selon la présente invention permettent la libération contrôlée d'au moins une substance active. Par libération contrôlée, on entend un profil de libération de la substance active souhaitable pour le traitement envisagé. La libération de la substance active peut donc être plus ou moins retenue ou ralentie en fonction de la substance active utilisée et de l'effet thérapeutique recherché. On notera que le contrôle de la libération de la substance active peut être aisément obtenu par de simples variations des proportions des composants des compositions selon la présente invention. Elles se prêtent donc très bien à diverses applications thérapeutiques dans lesquelles on recherche la libération contrôlée d'une substance active dans un site biologique bien précis.

Les compositions selon la présente invention sont des compositions pharmaceutiques fluides se présentant sous la forme d'émulsions, de suspensions ou de préparations huileuses. Elles possèdent la propriété de se gélifier instantanément en présence d'une phase aqueuse. En effet, lorsque les compositions selon la présente invention sont placées en présence d'un excès de phase aqueuse, elles passent d'un état fluide à un état de gel non miscible avec la phase aqueuse environnante.

Ceci rend les compositions de l'invention particulièrement bien adaptées pour le traitement de diverses affections nécessitant un traitement local dans un milieu irrigué en permanence par une phase aqueuse. De telles applications incluent notamment le traitement des parodontites, des gingivites, des abcès dentaires ou encore le traitement des aphtes et des mycoses.

Les préparations selon l'invention peuvent aussi être judicieusement utilisées pour obtenir, par les voies sous-cutanée et intra-musculaire, une libération soutenue et programmée de certains médicaments. Au contact de l'eau, un gel va se former sous la peau ou dans le muscle et le médicament pourra diffuser et être libéré lentement à partir de ce gel.

Selon un autre aspect, la présente invention concerne des procédés de préparation des compositions selon la présente invention. Les compositions selon la présente invention sont obtenues par un procédé comportant les étapes successives suivantes:
i) le ou les phospholipides sont dissous dans le ou les solvants pharmaceutiquement acceptables;
ii) le ou les acides gras sont ajoutés à la solution de phospholipide sous agitation;
iii) la ou les substances actives sont incorporées au mélange obtenu à la fin de l'étape ii), et
iv) de l'eau est éventuellement ajoutée à la composition obtenue à l'étape iii).

Lorsque la substance active est hydrosoluble, elle est dissoute dans une quantité minimale d'eau avant l'incorporation à l'étape iii). Lorsque la substance active n'est pas soluble dans l'eau, elle est incorporée à l'étape iii) dans le mélange de phospholipide, de solvant pharmaceutiquement acceptable et d'acide gras. Dans le cas d'une substance à la fois non hydrosoluble et peu ou pas liposoluble, elle est également incorporée à l'étape iii), éventuellement sous forme micronisée.

Les exemples qui suivent illustrent la présente invention sans toutefois la limiter. Dans ces exemples, toutes les parties sont exprimées en poids. Les produits commerciaux suivants ont été obtenus chez NATTERMANN PHOSPHOLIPID GmbH et possèdent les compositions suivantes (pourcentages en poids):
- Phospholipon 90™ : phosphatidylcholine;
- Phosal 50 PG™ : 55,8% de phosphatidylcholine, 1,9% d'acides gras de soja, 2,9% de monoglycérides de tournesol, 1,9% d'éthanol, 37,3% de propylène glycol, 0,2% de palimitate d'ascorbyle;
- NAT 8449™ : 60% de phosphatidylcholine, 40% de propylène glycol;
- Phosal 53 MCT™ : 60,8% de phosphatidylcholine, 2% d'acide oléique, 3% de monoglycérides de tournesol, 5% d'éthanol, 29% de triglycérides, 0,2% de palmitate d'ascorbyle;
- Phospholipon G-Na™ : sel sodique de 3(3-sn-phosphatidyl)glycérol de soja;
- Phospholipon CC™ : 1,2-dicaproyl-sn-glycéro(3)phosphocholine;
- Phospholipon SG-Na™ : sel sodique de 1,2-distéaroyl-sn-glycéro(3)phospholglycérol;
- Phospholipon 90 H™ : (3-sn-phosphatidyl)choline de soja hydrogénée.

### Exemple 1.

Cet exemple illustre la préparation de diverses compositions selon l'invention. Les compositions décrites ci-après se présentent sous la forme d'émulsions, suspensions ou solutions plus ou moins visqueuses qui gélifient instantanément en présence d'une phase aqueuse.

### Mode opératoire général a:

Le Phosal 50 PG™ ou le NAT 8449™ et l'acide oléique sont mélangés sous agitation. La substance active est introduite dans le mélange sous agitation. Après homogénéisation, on ajoute éventuellement de l'eau pour rendre la préparation plus visqueuse.

### Mode opératoire général b:

Le Phosal 50 PG™ ou le NAT 8449™ et l'acide oléique sont mélangés sous agitation. La substance active est dissoute dans l'eau, et la solution ainsi obtenue est introduite dans le mélange Phosal 50 PG™ ou NAT 8449™ - acide oléique sous agitation.

### 1.1. Préparation au benzoate de métronidazole.

Les préparations ayant les compositions présentées dans le Tableau 1 sont obtenues suivant le mode opératoire général a.

**Tableau 1 -**

| Compositions A et B - Métronidazole (parties) | | | | | |
|---|---|---|---|---|---|
| Composition | A₁ | A₂ | A₃ | B₁ | B₂ |
| Phosal 50 PG™ | 54,6 | 77,4 | 81,9 | - | - |
| NAT 8449™ | - | - | - | 72,8 | 45,5 |
| acide oléique | 36,4 | 13,6 | 9,1 | 18,2 | 45,5 |
| benzoate de metrodinazole | 5,0 | 5,0 | 5,0 | 5,0 | 5,0 |
| eau | 4,0 | 4,0 | 4,0 | 4,0 | 4,0 |

### 1.2 Préparation au diacétate de chlorhexidine.

Les préparations ayant les compositions présentées dans le Tableau 2 sont obtenues suivant le mode opératoire général a.

**Tableau 2 -**

| Compositions C et D - chlorhexidine (parties) | | | | |
|---|---|---|---|---|
| Composition | C₁ | C₂ | D₁ | D₂ |
| Phosal 50 PG™ | 51,0 | 63,8 | - | - |
| NAT 8449™ | - | - | 59,5 | 51,0 |
| acide oléique | 34,0 | 21,2 | 25,5 | 34,0 |
| diacétate de chlorhexidine | 15,0 | 15,0 | 15,0 | 15,0 |

### 1.3. Préparation à l'hyclate de doxycycline.

Les préparations ayant les compositions présentées dans le Tableau 3 sont obtenues suivant le mode opératoire général b.

**Tableau 3 -**

| Compositions E et F - Doxycycline (parties) | | | | |
|---|---|---|---|---|
| Composition | E₁ | E₂ | F₁ | F₂ |
| Phosal 50 PG™ | 43,0 | 64,5 | - | - |
| NAT 8449™ | - | - | 51,6 | 34,4 |
| acide oléique | 43,0 | 21,5 | 34,4 | 51,6 |
| hyclate de doxyxycline | 5,0 | 5,0 | 5,0 | 5,0 |
| eau | 9,0 | 9,0 | 9,0 | 9,0 |

### 1.4. Préparation au chlorhydrate de minocycline.

Les préparations ayant les compositions présentées dans le Tableau 4 sont obtenues suivant le mode opératoire général a.

**Tableau 4 -**

| Compositions G et H - Minocycline (parties) | | | | |
|---|---|---|---|---|
| Composition | G₁ | G₂ | H₁ | H₂ |
| Phosal 50 PG™ | 45,5 | 77,4 | - | - |
| NAT 8449™ | - | - | 68,3 | 45,5 |
| acide oléique | 45,5 | 13,6 | 22,7 | 45,5 |
| chlorhydrate de minocycline | 5,0 | 5,0 | 5,0 | 5,0 |
| eau | 4,0 | 4,0 | 4,0 | 4,0 |

### 1.5. Préparation à l'alcool 1,2-dichlorobenzylique

Les préparations ayant les compositions présentées dans le Tableau 5 sont obtenues suivant le mode opératoire général a.

**Tableau 5 -**

| Compositions I et J - Alcool 1,2-dichlorobenzylique (parties) | | |
|---|---|---|
| Composition | I | J |
| Phosal 50 PG™ | 80 | - |
| NAT 8449™ | - | 80 |
| acide oléique | 19 | 19 |
| alcool 1,2-dichlorobenzylique | 1 | 1 |

### 1.6. Préparation au succinate d'hydrocortisone.

Les préparations ayant les compositions présentées dans le Tableau 6 sont obtenues suivant le mode opératoire général b.

**Tableau 6 -**

| Compositions K et L - Hydrocortisone (parties) | | |
|---|---|---|
| Composition | K | L |
| Phosal 50 PG™ | 80 | - |
| NAT 8449™ | - | 67,0 |
| acide oléique | 15 | 28,0 |
| succinate d'hydrocortisone | 1 | 1 |
| eau | 4 | 4 |

### 1.7. Préparation au chlorhydrate de lidocaïne.

Les préparations ayant les compositions présentées dans le Tableau 7 sont obtenues suivant le mode opératoire général b.

**Tableau 7 -**

| Compositions M et N - Lidocaïne (parties) | | |
|---|---|---|
| Composition | M | N |
| Phosal 50 PG™ | 80 | - |
| NAT 8449™ | - | 66 |
| acide oléique | 14 | 28 |
| chlorhydrate de lidocaïne | 2 | 2 |
| eau | 4 | 4 |

### Exemple 2. Tests de libération.

Les préparations A₂ et B₁ préparées à l'exemple 1 ont été soumises à un test de libération effectué selon les normes de la 23 ème édition de la pharmacopée U.S. (USP 23), utilisant l'appareil n° 1 à une température de 37°C, les pales tournant à 50 tpm.

Ce test a montré que la préparation A₂ libère environ 60% du principe actif en 6 heures, la libération se poursuivant ensuite lentement pour atteindre environ 65% en 24 heures. Quant à la préparation B₁, elle libère environ 45% du principe actif en 6 heures, puis la libération continue lentement pour atteindre environ 55% en 24 heures.

### Exemple 3.

Cet exemple montre que différents solvants pharmaceutiquement acceptables peuvent être utilisés dans les compositions selon la présente invention. 3.1. Composition O : du Phospholipon 90™ (30 parties en poids) est dissous à chaud dans le polyéthylène glycol 400 (45 parties en poids). Après refroidissement, l'acide oléique est ajouté sous agitation. Au contact d'une solution aqueuse, la préparation gélifie instantanément.

Cet exemple montre que le propylène glycol peut être remplacé par du PEG 400.

3.2. Compositions P : on mélange sous agitation 40,9 parties de NAT 8449™, 27,3 parties de PEG 400, 22,8 parties en poids d'acide oléique. De l'eau (9 parties en poids) est ajoutée sous agitation pour rendre la préparation plus visqueuse.

Les préparations ayant les compositions présentées dans le Tableau 8 sont obtenues suivant ce mode opératoire.

**Tableau 8 -**

| Compositions P (parties) | | | |
|---|---|---|---|
| Composition | P₁ | P₂ | P₃ |
| NAT 8449™ | 34,1 | 40,9 | 61,4 |
| PEG 400 | 34,1 | 27,3 | 6,8 |
| acide oléique | 22,8 | 22,8 | 22,8 |
| eau | 9,0 | 9,0 | 9,0 |

### Exemple 4.

Cet exemple montre que les compositions selon la présente invention peuvent également contenir des triglycérides.

Composition Q: On mélange sous agitation 61,2 parties de Phosal 50 PG™, 20,4 parties de Phosal 53 MCT™ et 14,4 parties d'acide oléique. On ajoute ensuite 4 parties d'eau à ce mélange sous agitation.

Cette préparation gélifie instantanément au contact d'une phase aqueuse.

### Exemple 5.

Cet exemple montre que les compositions selon la présente invention peuvent contenir différents types de phospholipides. Les phospholipides utilisés sont le sel sodique du 3-(3-sn-phosphatidyl)glycérol de soja (Phospholipon G-Na™), la 1,2-dicaproyl-sn-glycéro(3)phosphocholine (Phospholipon CC™), le sel sodique du 1,2-distéaroyl-sn-glycéro (3) phosphoglycérol (Phospholipon SG-Na™) et la (3-sn-phosphatidyl)choline de soja hydrogénée (Phospholipon 90H™)

Les compositions P présentées dans le Tableau 9 sont obtenues en mélangeant les divers composants sous agitation. Ces quatre compositions gélifient instantanément en présence d'une phase aqueuse.

**Tableau 9 -**

| Compositions R (parties) | | | | |
|---|---|---|---|---|
| Composition | R₁ | R₂ | R₃ | R₄ |
| Phospholipon G-Na™ 30 | - | - | - | |
| Phospholipon CC™ | - | 30 | - | - |
| Phospholipon SG-Na™ | - | - | 15 | - |
| Phospholipon 90H™ | - | - | - | 3 |
| PEG 400 | 45 | 45 | 60 | 72 |
| acide oléique | 25 | 25 | 25 | 25 |

### Exemple 6.

Cet exemple montre que l'acide oléique peut être remplacé par d'autres acides gras ou par un alcool gras dans les compositions selon la présente invention.

Les compositions S présentées dans le Tableau 10 sont obtenues en mélangeant les divers composants sous agitation. Ces quatre compositions gélifient instantanément en présence d'une phase aqueuse.

**Tableau 10 -**

| Compositions S (parties) | | | | |
|---|---|---|---|---|
| Composition | S₁ | S₂ | S₃ | S₄ |
| Phosal 50PG™ | 80 | 80 | 80 | 80 |
| acide caprylique | 20 | - | - | - |
| acide caprique | - | 20 | - | - |
| acide oléique | - | - | 20 | - |
| alcool oléique | - | - | - | 20 |

### Exemple 7. Mesure de la vitesse de libération en fonction des excipients.

7.1. Les compositions T présentées dans le Tableau 10 sont obtenues en ajoutant la quantité voulue d'une solution aqueuse à 10% de colorant Sicomet-FDC bleu 1 au mélange des autres composants sous agitation.
Les compositions T₁ à T₆ gélifient instantanément en présence d'une phase aqueuse.

**Tableau 11 -**

| Compositions T (parties) | | | | | | | |
|---|---|---|---|---|---|---|---|
| Composition | contrôle | T₁ | T₂ | T₃ | T₄ | T₅ | T₆ |
| Phosal 50PG™ | - | 81,6 | 68,3 | 68,3 | 68,3 | 68,3 | 68,3 |
| acide oléique | - | 14,4 | 22,7 | 18,2 | 13,7 | 18,2 | 9,0 |
| Miglyol 810N™ | - | - | - | 4,5 | 9,0 | - | 13,7 |
| Phosal 53 MCT™ | - | - | - | - | - | 4,5 | - |
| solution de colorant | 100 | 4,0 | 9,0 | 9,0 | 9,0 | 9,0 | 9,0 |

Le test de libération est effectué comme suit. Des quantités égales des préparations T₁ à T₆ et de la solution de contrôle sont déposées dans un puits creusé au centre d'une couche d'épaisseur constante de trypticase soy agar coulée dans une boîte de Pétri. La vitesse de diffusion du colorant est déterminée en mesurant le diamètre de la tache de colorant en fonction du temps. Les résultats obtenus pour la solution de contrôle et les solutions T₁ à T₆ sont repris dans le Tableau 12.

**Tableau 12 -**

| Vitesse de libération des préparations T1 à T6. | | | | | | | |
|---|---|---|---|---|---|---|---|
| Temps (heures) | Diamètre de la tache en mm. | | | | | | |
| | contrôle | T₁ | T₂ | T₃ | T₄ | T₅ | T₆ |
| 0 | 16,97 | 18,74 | 18,61 | 17,66 | 18,36 | 18,49 | * |
| 3 | 49,55 | - | - | - | - | - | * |
| 6 | 62,18 | 29,56 | 29,14 | 28,58 | 23,12 | 33,33 | * |
| 24 | 90,10 | 51,00 | 30,38 | 30,57 | 24,59 | 52,96 | * |
| 36 | 96,29 | 57,45 | 34,02 | 33,67 | 31,23 | 54,55 | * |
| 72 | 108,52 | 60,45 | 39,29 | 34,58 | 31,82 | 68,67 | * |

| | | | | | | | |
|---|---|---|---|---|---|---|---|
| * Aucune diffusion n'est observée. | | | | | | | |

Cet exemple montre que l'on peut contrôler la vitesse de libération d'une substance active par le choix des composants de la préparation.
7.2. De manière analogue, on a préparé les compositions U reprises dans le Tableau 13 et constaté que la diffusion du colorant diminue avec la teneur en acide oléique.

**Tableau 13 -**

| Compositions U (parties) | | | |
|---|---|---|---|
| Composition | U₁ | U₂ | U₃ |
| Phosal 50PG™ | 81,6 | 86,4 | 91,2 |
| acide oléique | 14,4 | 9,6 | 4,8 |
| solution de colorant | 4,0 | 4,0 | 4,0 |

### Exemple 8. Test clinique

L'efficacité des compositions selon la présente invention pour le traitement des parodontites a été évaluée dans un test clinique, dans lequel la préparation A₂ contenant 5% de benzoate de métronidazole a été injectée dans les poches périodontales de patients soufrant de parodontites.

Le test comportait deux volets: une étude visant à mesurer la vitesse de, diffusion de l'acide benzoïque et du métronidazole dans le fluide gingival et une étude médicale visant à évaluer l'état d'avancement de la guérison des patients au cours du temps.

Juste avant le démarrage du test, la plaque sus-gingivale des patients est éliminée à la pointe à ultrasons, les dents sont isolées par des rouleaux de coton, et les sites traités sont rincés à l'eau et délicatement séchés par jet d'air. Le site traité est alors rempli par la suspension active à l'aide d'une seringue sur laquelle une aiguille à bout mousse a été placée.

Pour les mesures de la vitesse de diffusion de l'acide benzoïque et du métronidazole, des échantillons de fluide gingival sont collectés à différents intervalles de temps après remplissage du site, à l'aide de languettes de 2 x 13 mm de papier Whatmann n°4 introduites à une profondeur de 1 à 2 mm dans la partie crévicale de la poche et maintenues pendant 120 secondes dans cette position pour permettre l'absorption du fluide. Les papiers filtres sont conservés à -10°C dans des tubes stériles de polypropylène.

Les quantités de benzoate de métronidazole dans le fluide gingival ont été déterminées par HPLC (appareil WATERS™ utilisant un détecteur UV WATERS™ 486, une colonne CHOMOSPHER™ 5µm C18 et, comme phase mobile, un mélange d'eau (tamponnée avec du nitrate de potassium et de l'acétate de sodium dont le pH a été ajusté à 5,5 au moyen d'acide acétique) et de méthanol 40/60 vol/vol; débit 1 ml/min).

Les résultats des dosages, exprimés en µg de métronidazole par ml de fluide créviculaire en fonction du temps, sont repris dans le Tableau 14.

**Tableau 14 -**

| Préparation A2: concentrations moyennes exprimées en métronidazole en fonction du temps | | |
|---|---|---|
| Temps (h) | Concentration movenne (µg/ml) | Erreur type de la moyenne |
| 0,5 | 177,03 | 56,0 |
| 5,5 | 13,48 | 2,2 |
| 9,5 | 3,35 | 1,1 |
| 25 | 16,38 | 1,1 |
| 50 | 16,67 | 1,9 |
| 85 | 18,39 | 1,3 |
| 135 | 14,07 | 0,8 |
| 200 | 14,29 | 0,8 |

Les résultats obtenus montrent que la concentration calculée en métronidazole dans le fluide créviculaire est, pendant une durée supérieure à 8 jours, très largement supérieure à la concentration minimale inhibitrice (MIC) des germes pathogènes anaérobies sensibles au métronidazole de 1,75 à 5 µg/ml

L'Elysol™, une préparation de benzoate de métronidazole dosée à 25% en métronidazole, donne pour sa part, selon K. Stolze (J. Clin. Periodontol., 19, 698-701 (1992)), des concentrations dans le fluide créviculaire reprises dans le Tableau 15.

**Tableau 15 -**

| Elysol : Concentrations moyennes exprimées en fonction du temps | | |
|---|---|---|
| Temps (h) | Concentration moyenne (µg/ml) | Erreur type de la moyenne |
| 12 | 30,0 | 7,6 |
| 24 | 8,5 | 5,4 |
| 36 | 1,3 | 1,0 |

Après 36 heures, la concentration exprimée en métronidazole est inférieure à la MIC.

## Revendications

1. Utilisation d'une composition pharmaceutique fluide permettant la libération contrôlée d'au moins une substance active comprenant
a) une quantité thérapeutiquement efficace d'au moins une substance active,
b) de 3 à 55% en poids de phospholipide,
c) de 16 à 72% en poids de solvant pharmaceutiquement acceptable, et
d) de 4 à 52% en poids d'acide gras,
ladite composition ayant la propriété de se gélifier instantanément en présence d'une phase aqueuse, pour la préparation d'un médicament destiné au traitement d'une personne ou d'un animal soufrant de parodontite.

2. Utilisation d'une composition pharmaceutique selon la revendication 1, **caractérisée en ce que** la substance active est choisie parmi les antibiotiques, les agents anti-infectieux, les anesthésiques locaux, les anti-inflammatoires, les antimycosiques ou des substances actives peptidiques.

3. Utilisation d'une composition pharmaceutique selon la revendication 1 ou 2, **caractérisée en ce que** le phospholipide est choisi parmi la phosphatidylcholine, les sels du phosphatidylglycérol, la dicaproylphosphatidylcholine et les sels du distéaroylphosphatidylglycérol, seuls ou en mélange.

4. Utilisation d'une composition pharmaceutique selon la revendication 3, **caractérisée en ce que** la composition pharmaceutique contient de 15 à 55% en poids de phospholipide, de préférence, de 15 à 51% en poids de phospholipide.

5. Utilisation d'une composition pharmaceutique selon la revendication 1 ou 2, **caractérisée en ce que** le phospholipide est une phosphatidylcholine hydrogénée.

6. Utilisation d'une composition selon la revendication 5, **caractérisée en ce que** la composition contient de 3 à 11%, de préférence de 3 à 10% en poids de phospholipide.

7. Utilisation d'une composition selon l'une quelconque des revendications 1 à 6, **caractérisée en ce que** le solvant pharmaceutiquement acceptable est choisi parmi le propylène glycol, les polyéthylène glycols ou les huiles minérales telles que l'huile de paraffine ou les huiles de silicone, seuls ou en mélange.

8. Utilisation d'une composition selon l'une quelconque des revendications 1 à 7, **caractérisée en ce que** les acides gras présents dans ladite composition sont des acides carboxyliques organiques saturés ou insaturés contenant de 4 à 22 atomes de carbone, de préférence de 8 à 18 atomes de carbone.

9. Utilisation d'une composition pharmaceutique selon la revendication 8, **caractérisée en ce que** les acides gras sont choisis parmi l'acide oléique, l'acide caprylique, l'acide caprique, l'acide caproïque, l'acide myristique ou l'acide butyrique, seuls ou en mélange.

10. Utilisation d'une composition pharmaceutique selon l'une quelconque des revendications 1 à 9, **caractérisée en ce que** la composition comprend en outre jusqu'à 5% en poids de monoglycéride ou de diglycéride ou d'un mélange de mono- et de diglycéride et/ou jusqu'à 15% en poids de triglycérides.

## Patentansprüche

1. Verwendung einer flüssigen pharmazeutischen Zusammensetzung, die die kontrollierte Freisetzung von wenigstens einer aktiven Substanz erlaubt, die
a) eine therapeutisch wirksame Menge wenigstens eines Wirkstoffs,
b) 3 bis 55 Gew.-% Phospholipid,
c) 16 bis 72 Gew.-% pharmazeutisch annehmbares Lösungsmittel und
d) 4 bis 52 Gew.-% Fettsäure
umfasst, wobei besagte Zusammensetzung die Eigenschaft hat, in Gegenwart einer wässrigen Phase sofort zu gelieren, für die Herstellung eines Medikaments, das zur Behandlung einer an Parodontose leidenden Person oder eines Tiers bestimmt ist.

2. Verwendung einer pharmazeutischen Zusammensetzung gemäß Anspruch 1, **dadurch gekennzeichnet, dass** der Wirkstoff unter den Antibiotika, den Antiinfektiosa, den Lokalanästhetika, den Antientzündungsmitteln, den Antimykotika oder den peptidischen Wirkstoffen ausgewählt ist.

3. Verwendung einer pharmazeutischen Zusammensetzung gemäß Anspruch 1 oder 2, **dadurch gekennzeichnet, dass** das Phospholipid unter Phosphatidylcholin, den Phosphatidylglycerol-Salzen, Dicaproylphosphatidylcholin und den Distearoylphosphatidylglycerol-Salzen, allein oder im Gemisch, ausgewählt ist.

4. Verwendung einer pharmazeutischen Zusammensetzung gemäß Anspruch 3, **dadurch gekennzeichnet, dass** die pharmazeutische Zusammensetzung 15 bis 55 Gew.-% Phospholipid, vorzugsweise 15 bis 51 Gew.-% Phospholipid enthält.

5. Verwendung einer pharmazeutischen Zusammensetzung gemäß Anspruch 1 oder 2, **dadurch gekennzeichnet, dass** das Phospholipid ein hydriertes Phosphatidylcholin ist.

6. Verwendung einer Zusammensetzung gemäß Anspruch 5, **dadurch gekennzeichnet, dass** die Zusammensetzung 3 bis 11 Gew.-%, vorzugsweise 3 bis 10 Gew.-% Phospholipid enthält.

7. Verwendung einer Zusammensetzung gemäß einem der Ansprüche 1 bis 6, **dadurch gekennzeichnet, dass** das pharmzeutisch annehmbare Lösungsmittel unter Propylenglycol, den Polyethylenglycolen oder solchen Mineralölen wie Paraffinöl oder Silikonölen, allein oder im Gemisch, ausgewählt sind.

8. Verwendung einer Zusammensetzung gemäß einem der Ansprüche 1 bis 7, **dadurch gekennzeichnet, dass** die in besagter Zusammensetzung vorhandenen Fettsäuren gesättigte oder ungesättigte organische Carbonsäuren mit 4 bis 22 Kohlenstoffatomen, vorzugsweise mit 8 bis 18 Kohlenstoffatomen sind.

9. Verwendung einer pharmazeutischen Zusammensetzung gemäß Anspruch 8, **dadurch gekennzeichnet, dass** die Fettsäuren unter Oleinsäure, Caprylsäure, Capronsäure, Caprinsäure, Myristinsäure oder Buttersäure, allein oder im Gemisch, ausgewählt sind.

10. Verwendung einer pharmazeutischen Zusammensetzung gemäß einem der Ansprüche 1 bis 9, **dadurch gekennzeichnet, dass** die Zusammensetzung außerdem bis zu 5 Gew.-% Monoglycerid oder Diglycerid oder eines Gemisches aus Mono- und Diglycerid und/oder bis zu 15 Gew.-% Triglyceride umfasst.

## Claims

1. Use of a fluid pharmaceutical composition which allows the controlled release of at least one active substance and which comprises
a) a therapeutically effective amount of at least one active substance,
b) from 3 to 55% by weight of phospholipid,
c) from 16 to 72% by weight of pharmaceutically acceptable solvent, and
d) from 4 to 52% by weight of fatty acid,
said composition having the property of gelling instantaneously in the presence of an aqueous phase, for preparing a medicinal product intended for treating a person or an animal suffering from periodontitis.

2. Use of a pharmaceutical composition according to claim 1, **characterized in that** the active substance is chosen from antibiotics, anti-infectious agents, local anesthetics, antiinflammatory agents, anti-mycotic agents and peptide active substances.

3. Use of a pharmaceutical composition according to either of claims 1 and 2, **characterized in that** the phospholipid is chosen from phosphatidylcholine, phosphatidylglycerol salts, dicaproylphosphatidylcholine and distearoylphosphatidylglycerol salts, alone or as a mixture.

4. Use of a pharmaceutical composition according to claim 3, **characterized in that** the pharmaceutical composition contains from 15 to 55% by weight of phospholipid, preferably from 15 to 51% by weight of phospholipid.

5. Use of a pharmaceutical composition according to either of claims 1 and 2, **characterized in that** the phospholipid is a hydrogenated phosphatidylcholine.

6. Use of a composition according to claim 5, **characterized in that** the composition contains from 3 to 11%, preferably from 3 to 10%, by weight of phospholipid.

7. Use of a composition according to any one of claims 1 to 6, **characterized in that** the pharmaceutically acceptable solvent is chosen from propylene glycol, polyethylene glycols and mineral oils such as liquid paraffin or silicone oils, alone or as a mixture.

8. Use of a composition according to any one of claims 1 to 7, **characterized in that** the fatty acids present in said composition are saturated or unsaturated organic carboxylic acids containing from 4 to 22 carbon atoms, preferably from 8 to 18 carbon atoms.

9. Use of a pharmaceutical composition according to claim 8, **characterized in that** the fatty acids are chosen from oleic acid, caprylic acid, capric acid, caproic acid, myristic acid and butyric acid, alone or as a mixture.

10. Use of a pharmaceutical composition according to any one of claims 1 to 9, **characterized in that** the composition also comprises up to 5% by weight of monoglyceride or of diglyceride or of a mixture of mono- and of diglyceride, and/or up to 15% by weight of triglycerides.
